# EUROPEAN PATENT APPLICATION

(11) **EP 4 592 333 A1**
(43) Date of publication of application: **30.07.2025**
(21) Application number: 23868163.9
(22) Date of filing: 19.09.2023
(51) Int. Cl.: C08J 3/12, A61K 8/85, A61Q 1/12, C08J 3/16, C08L 67/04, C09D 167/04

(54) **RESIN PARTICLES AND USE THEREOF**

(30) Priority: 21.09.2022 JP 2022149671
(71) Applicant: Sekisui Kasei Co., Ltd., Kita-ku Osaka-shi Osaka 530-0047 (JP)
(72) Inventor: MOTOMURA, Takashi, Osaka-shi, Osaka 530-0047 (JP); KOBAYASHI, Hironori, Osaka-shi, Osaka 530-0047 (JP); MUKAI, Ken, Osaka-shi, Osaka 530-0047 (JP)
(74) Representative: Müller-Boré & Partner Patentanwälte PartG mbB
(86) International application number: PCT/JP2023/033863
(87) International publication number: WO 2024/063042

(57) **Abstract**

An object is to provide a polyhydroxyalkanoate-based resin particle in which an odor is suppressed. As a solution, the following (i) to (iii); (i) a polyhydroxyalkanoate-based resin particle in which a total remaining amount of an unsaturated fatty acid is 10 mass ppm or less, (ii) a polyhydroxyalkanoate-based resin particle in which a total remaining amount of crotonic acid is 10 mass ppm or less, and (iii) a polyhydroxyalkanoate-based resin particle in which a total remaining amount of a pentenoic acid is 60 mass ppm or less are provided.

## Description

### [Technical Field]

The present invention relates to a resin particle and an application thereof. In detail, the present invention relates to a resin particle, a topical preparation containing a resin particle, a coating material containing a resin particle, a resin composition containing a resin particle, and an anti-blocking agent containing a resin particle.

### [Background Art]

Since the specific surface area, particle shape, and particle structure of a resin particle can be arbitrarily adjusted, modification, physical property improvement, and the like are performed by blending the resin particle into a variety of materials, articles, and the like. Examples of the main applications of the resin particle include applications such as compounding agents for cosmetics such as foundations, antiperspirants, and scrubbing agents, matting agents for coating materials (paints), a variety of agents such as rheology modifiers, anti-blocking agents, slipperiness imparting agents, light diffusing agents, medical diagnostic testing agents, and additives for molded products such as automotive materials and building materials. Examples of the resin particle include those of urethane, acrylic, silicone, polyethylene, and the like.

Incidentally, amid the increasing interest in environmental issues in recent years, there has been a demand for the use of non-petroleum raw material-derived materials or biodegradable materials in all fields where resins are used to reduce environmental impact, such as the microplastics issue. For example, there has been such a demand even in fields where resin particles are used, such as cosmetics and coating materials (paints).

Under such circumstances, a variety of biodegradable resin particles are known.

PTL 1 describes a cellulose acetate particle in which the average particle diameter is 80 nm or more and 100 µm or less, the sphericity is 0.7 or more and 1.0 or less, the surface smoothness is 80% or more and 100% or less, and the total acetyl substitution degree of cellulose acetate is 0.7 or more and 2.9 or less. It is described that this cellulose acetate particle has excellent biodegradability and texture and can be used by being blended into cosmetic compositions.

PTL 2 describes an aliphatic polyester-based resin particle in which the volume average particle diameter is 2 to 30 µm, the proportion of a particle having a particle diameter of less than 1 µm is 15 vol% or less, and the proportion of a particle having a particle diameter of more than 30 µm is 6 vol% or less. It is described that a cosmetic containing this aliphatic polyester-based resin particle is environmentally friendly and is excellent in terms of spreadability, adhesion, texture (the particle has neither a foreign-material sensation nor squeakiness but has a soft sensation, slipperiness, and smoothness) and transparency upon application.

PTL 3 describes a polyhydroxyalkanoate particle obtained by crushing an aggregate of primary particles. This polyhydroxyalkanoate particle can be used as a matting agent for coating compositions and is considered to be capable of making coatings more favorable than or comparable to conventional coatings containing a petrochemical raw material-based matting agent while reducing the proportion of a petrochemical substance.

PTL 4 describes a biodegradable resin particle made of a polyhydroxyalkanoate, in which the amount of a Ca component in the particle is 10 to 10,000 ppm, the volume average particle diameter is 2 to 50 µm, the BET specific surface area is 0.8 to 10 m²/g, and the linseed oil absorption is 50 to 300 mL/100 g. It is considered that this biodegradable resin particle has a small particle diameter, is excellent in terms of adhesion to skin and easy spread on skin in the case of being applied to skin, and can be suitably used by being blended into topical preparations such as cosmetics and quasi-drugs.

### [Citation List]

### [Patent Literature]

[PTL 1] Japanese Patent No. 6609726
[PTL 2] WO 2017/195642
[PTL 3] Japanese Patent No. 5671631
[PTL 4] WO 2020/262509

### [Summary of Invention]

### [Technical Problem]

A cellulose acetate particle has a strong squeaky sensation and has a problem with texture in the case of being blended into cosmetics. In addition, the cellulose acetate particle has another problem with an odor arising from a component that is generated by hydrolysis and is also not satisfactory in terms of biodegradability in water.

For the aliphatic polyester-based resin particle of PTL 2 and the biodegradable resin particles made of a polyhydroxyalkanoate of PTL 3 and 4, no studies have been conducted regarding the heat resistance and odors of the resin particles, and furthermore, the biodegradability of the resin particles in water. Furthermore, since the polyhydroxyalkanoate particle of PTL 3 is obtained by crushing with a jet mill, the particle shape is not stable, and the texture upon application is not satisfactory when the particle is blended into cosmetics.

A first problem that the present invention is intended to solve is to provide a polyhydroxyalkanoate-based resin particle in which an odor is suppressed.

A second problem that the present invention is intended to solve is to provide a polyhydroxyalkanoate-based resin particle in which an odor is suppressed, biodegradability in water is excellent, and there is little squeaky sensation.

A third problem that the present invention is intended to solve is to provide a polyhydroxyalkanoate-based resin particle in which an odor is suppressed, biodegradability in water is excellent, there is little squeaky sensation, and dispersibility in a topical preparation is excellent.

A fourth problem that the present invention is intended to solve is to provide one or more of a topical preparation containing the polyhydroxyalkanoate-based resin particle, a coating material containing the polyhydroxyalkanoate-based resin particle, a resin composition containing the polyhydroxyalkanoate-based resin particle, and an anti-blocking agent containing the polyhydroxyalkanoate-based resin particle.

### [Solution to Problem]

As a result of intensive studies for solving the above-described problems, the present inventors found that the problems can be solved by a specific polyhydroxyalkanoate-based resin particle, and completed the present invention.

As the present invention, an invention for solving the first problem is inventions according to the following items 1 to 4.

As the present invention, an invention for solving the second problem is an invention according to the following item 5.

As the present invention, an invention for solving the third problem is an invention according to the following item 6.

As the present invention, an invention for solving the fourth problem is inventions according to the following items 7 to 10.
[Item 1] A polyhydroxyalkanoate-based resin particle, in which a total remaining amount of an unsaturated fatty acid is 10 mass ppm or less.
[Item 2] A polyhydroxyalkanoate-based resin particle, in which a total remaining amount of crotonic acid is 10 mass ppm or less.
[Item 3] A polyhydroxyalkanoate-based resin particle, in which a total remaining amount of a pentenoic acid is 60 mass ppm or less.
[Item 4] The polyhydroxyalkanoate-based resin particle according to any one of the items 1 to 3, in which the polyhydroxyalkanoate-based resin particle contains a poly(3-hydroxybutyrate-co-3-hydroxyvalerate) resin.
[Item 5] The polyhydroxyalkanoate-based resin particle according to any one of the items 1 to 4, in which a volume average particle diameter is 1 µm or more and 40 µm or less.
[Item 6] The polyhydroxyalkanoate-based resin particle according to any one of the items 1 to 5, in which a moisture content is 0.2 mass% or more and 0.8 mass% or less.
[Item 7] A topical preparation containing the polyhydroxyalkanoate-based resin particle according to any one of the items 1 to 6.
[Item 8] A coating material containing the polyhydroxyalkanoate-based resin particle according to any one of the items 1 to 6.
[Item 9] A resin composition containing the polyhydroxyalkanoate-based resin particle according to any one of the items 1 to 6.
[Item 10] An anti-blocking agent containing the polyhydroxyalkanoate-based resin particle according to any one of the items 1 to 6.

### [Advantageous Effects of Invention]

The present invention provides a polyhydroxyalkanoate-based resin particle in which a total remaining amount of an unsaturated fatty acid is within a specific range, a polyhydroxyalkanoate-based resin particle in which a total remaining amount of crotonic acid is within a specific range, and a polyhydroxyalkanoate-based resin particle in which a total remaining amount of a pentenoic acid is within a specific range, and provides a polyhydroxyalkanoate-based resin particle in which an odor is suppressed.

The present invention provides a polyhydroxyalkanoate-based resin particle in which a total remaining amount of an unsaturated fatty acid and a volume average particle diameter are within specific ranges and provides a polyhydroxyalkanoate-based resin particle in which an odor is suppressed, biodegradability in water is excellent, and there is little squeaky sensation.

The present invention provides a polyhydroxyalkanoate-based resin particle in which a total remaining amount of an unsaturated fatty acid, a volume average particle diameter, and a moisture content are within specific ranges and provides a polyhydroxyalkanoate-based resin particle in which an odor is suppressed, biodegradability in water is excellent, there is little squeaky sensation, and dispersibility in a topical preparation is excellent.

The present invention provides a topical preparation containing a specific polyhydroxyalkanoate-based resin particle, a coating material containing a specific polyhydroxyalkanoate-based resin particle, a resin composition containing a specific polyhydroxyalkanoate-based resin particle, and an anti-blocking agent containing a specific polyhydroxyalkanoate-based resin particle.

### [Description of Embodiments]

Hereinafter, a resin particle of the present invention, a topical preparation containing the resin particle, a coating material containing the resin particle, a resin composition containing the resin particle, and an anti-blocking agent containing the resin particle will be described in detail.

In addition, in the present specification, numerical ranges can be ranges obtained by any combinations of given upper limit values and lower limit values.

### [Polyhydroxyalkanoate-Based Resin Particle]

In a polyhydroxyalkanoate-based resin particle in the present invention, the resin particle is substantially composed of a polyhydroxyalkanoate-based resin, and other components may be contained in the resin particle.

The resin particle may contain one polyhydroxyalkanoate-based resin singly or may contain two or more polyhydroxyalkanoate-based resins.

Examples of the other components include a variety of components that are used at the time of producing the resin particle or a variety of components that are added to improve the characteristics or the like of the resin particle.

### <Polyhydroxyalkanoate-Based Resin>

The polyhydroxyalkanoate-based resin that composes the polyhydroxyalkanoate-based resin particle of the present invention is a biodegradable resin including a repeating unit derived from an aliphatic hydroxycarboxylic acid and may be a homopolymer or a copolymer.

Examples of the aliphatic hydroxycarboxylic acid include one or more selected from the group consisting of 3-hydroxypropionate, 3-hydroxybutyrate, 3-hydroxyvalerate, 3-hydroxyhexanoate, 3-hydroxyheptanoate, 3-hydroxyoctanoate, 3-hydroxynonanoate, 3-hydroxydecanoate, 3-hydroxyundecanoate, 3-hydroxydodecanoate, 3-hydroxytetradecanoate, 3-hydroxyhexadecanoate, 3-hydroxyoctadecanoate, lactic acid, 4-hydroxybutyrate, 4-hydroxyvalerate, 5-hydroxyvalerate, 6-hydroxyhexanoate, and the like.

The polyhydroxyalkanoate-based resin is preferably a poly(3-hydroxyalkanoate)-based polymer or copolymer including a repeating unit represented by a general formula (1);

-[-CH(R)-CH₂CO-O-]- (1)

(here, R in the formula (1) is an alkyl group represented by - CₙH₂ₙ₊₁, and n is an integer of 1 to 15).

In the present invention, the polyhydroxyalkanoate-based resin preferably contains a 3-hydroxybutyrate unit as a main component (for example, 50 mol% or more and preferably 80 mol% or more). For example, one or more selected from the group consisting of a poly(3-hydroxybutyrate-co-3-hydroxyvalerate) resin that is a copolymer of a 3-hydroxybutyrate unit and a 3-hydroxyvalerate unit, a poly(3-hydroxybutyrate-co-3-hydroxyhexanoate) resin that is a copolymer of a 3-hydroxybutyrate unit and a 3-hydroxyhexanoate unit, and a poly(3-hydroxybutyrate-co-3-hydroxyoctanoate) resin that is a copolymer of a 3-hydroxybutyrate unit and a 3-hydroxyoctanoate unit are preferably contained as main components, and a poly(3-hydroxybutyrate-co-3-hydroxyvalerate) resin is more preferably contained as a main component.

When a resin including a 3-hydroxybutyrate unit as a main component (for example, 50 mol% or more and preferably 80 mol% or more), for example, a poly(3-hydroxybutyrate-co-3-hydroxyvalerate) resin, a poly(3-hydroxybutyrate-co-3-hydroxyhexanoate) resin, and a poly(3-hydroxybutyrate-co-3-hydroxyoctanoate) resin, or preferably, a poly(3-hydroxybutyrate-co-3-hydroxyvalerate) resin is contained as a main component in the polyhydroxyalkanoate-based resin, biodegradability, particularly, underwater degradability is excellent, the microplastics issue, which has become an issue recently, can be dealt with, and the particle has a specific color value, whereby it is easier to match the tint than normal white particles, and it is possible to make the particle less noticeable when the particle is used particularly as a compounding agent for a slipperiness imparting agent for a foundation or an antiperspirant as a cosmetic.

### <Total Remaining Amount of Unsaturated Fatty Acid, Crotonic Acid, or Pentenoic Acid>

In a polyhydroxyalkanoate-based resin particle according to one embodiment of the present invention, the total remaining amount of an unsaturated fatty acid relative to the entire polyhydroxyalkanoate-based resin particle is 10 mass ppm or less. The total remaining amount is preferably 8 mass ppm or less, more preferably 6 mass ppm or less, and still more preferably 5 mass ppm or less.

In the polyhydroxyalkanoate-based resin particle according to one embodiment of the present invention, the total remaining amount of crotonic acid relative to the entire polyhydroxyalkanoate-based resin particle is 10 mass ppm or less. The total remaining amount is preferably 8 mass ppm or less, more preferably 6 mass ppm or less, and still more preferably 5 mass ppm or less.

In the polyhydroxyalkanoate-based resin particle according to one embodiment of the present invention, the total remaining amount of a pentenoic acid relative to the entire polyhydroxyalkanoate-based resin particle is 60 mass ppm or less. The total remaining amount is preferably 30 mass ppm or less, more preferably 10 mass ppm or less, and still more preferably 5 mass ppm or less.

The polyhydroxyalkanoate-based resin is partially decomposed by heat or the like and thereby generates an unsaturated fatty acid. For example, unsaturated fatty acids, such as crotonic acid or the like from the 3-hydroxybutyrate unit in the polyhydroxyalkanoate-based resin, 2-pentenoic acid, 3-pentenoic acid, 4-pentenoic acid, or the like from the 3-hydroxyvalerate unit, 2-hexenoic acid or the like from the 3-hydroxyhexanoate unit, and 2-octenoic acid or the like from the 3-hydroxyoctanoate unit are generated by decomposition and contained in the polyhydroxyalkanoate-based resin particle. These unsaturated fatty acids emit unique odors, and there is thus a concern of a problem of an abnormal odor being imparted to topical preparations or the like in a case where the polyhydroxyalkanoate-based resin particle has been blended into the topical preparations or the like.

The total remaining amount of the unsaturated acids is measured by a method to be described in the following examples.

### <Volume Average Particle Diameter>

The volume average particle diameter of the polyhydroxyalkanoate-based resin particle of the present invention is not particularly limited. The volume average particle diameter is, for example, 1 µm or more, preferably 3 µm or more, more preferably 5 µm or more, and particularly preferably 6 µm or more and is, for example, 40 µm or less, preferably 33 µm or less, more preferably 30 µm or less, and particularly preferably 25 µm or less. In a case where the volume average particle diameter is less than 1 µm, a squeak is likely to be generated in a topical preparation containing the polyhydroxyalkanoate-based resin particle, and there is a concern that the texture upon the application of a topical preparation containing the polyhydroxyalkanoate-based resin particle (texture when the topical preparation is applied to skin) may become poor. In a case where the volume average particle diameter is more than 40 µm, there is a concern that the texture upon the application of a topical preparation containing the polyhydroxyalkanoate-based resin particle (texture when the topical preparation is applied to skin) may become poor.

The volume average particle diameter is measured by a method to be described in the following examples.

### <Moisture Content>

The moisture content of the polyhydroxyalkanoate-based resin particle of the present invention is not particularly limited. The moisture content is, for example, 0.2 mass% or more and preferably 0.3 mass% or more and is, for example, 0.8 mass% or less and preferably 0.7 mass% or less. In a case where the moisture content is less than 0.2 mass% and a case where the moisture content is more than 0.8 mass%, there is a concern that it may not be possible to uniformly disperse the polyhydroxyalkanoate-based resin particle in a topical preparation in a case where the polyhydroxyalkanoate-based resin particle has been added to the topical preparation.

Here, the moisture content of the polyhydroxyalkanoate-based resin particle represents the content of water in the polyhydroxyalkanoate-based resin particle when the polyhydroxyalkanoate-based resin particle is set to 100 mass%.

The moisture content can be measured by, for example, a method to be described in the following examples.

### <Average Circularity>

The average circularity of the polyhydroxyalkanoate-based resin particle of the present invention is not particularly limited. The average circularity is, for example, 0.75 or more, preferably 0.78 or more, and more preferably 0.8 or more, and is for example, 1.0 or less, and preferably 0.99 or less. When the average circularity is set to 0.8 or more, it is possible to make the texture upon the application of a topical preparation containing the polyhydroxyalkanoate-based resin particle excellent.

The average circularity is measured by a method to be described in the following examples.

### <Color Tone>

The color tone of the polyhydroxyalkanoate-based resin particle of the present invention is not particularly limited.

The L* value in the L*a*b* color space is, for example, 70 or more, preferably 75 or more, and more preferably 80 or more.

The a* value in the L*a*b* color space is, for example, - 3.0 or more, preferably -2.5 or more, and more preferably -2.0 or more and is, for example, +2.0 or less, preferably +1.5 or less, and more preferably +1.0 or less.

The b* value in the L*a*b* color space is, for example, +1.0 or more, preferably +1.5 or more, and more preferably +2.0 or more and is, for example, +7.0 or less, preferably +6.5 or less, and more preferably +6.0 or less.

It is preferable that in the L*a*b* color space, the L* value is 70 or more, the a* is -3.0 or more and +2.0 or less, and the b* value is +1.0 or more and +7.0 or less, and it is more preferable that in the L*a*b* color space, the L* value is 80 or more, the a* is -2.0 or more and +1.0 or less, and the b* value is +2.0 or more and +6.0 or less.

The L*a*b* color space (L*a*b* color space) is a color space that was standardized by the International Commission on Illumination (CIE) in 1976 and was adopted in Japan by JIS Z 8781-4. The L*a*b* color space is a color space that is generally used at the time of representing the color of an object.

In the L*a*b* color space, the L* value is an index that indicates the brightness of color. As the L* value approaches 100, the color becomes brighter (white), and as the L* value approaches 0, the color becomes darker (black).

In the L*a*b* color space, the a* value is an index that indicates the intensity of red and green hues. The a* value indicates a redder hue in the positive direction, and indicates a greener hue in the negative direction.

In the L*a*b* color space, the b* value is an index that indicates the intensity of yellow and blue hues. The b* value indicates a yellower hue in the positive direction, and indicates a bluer hue in the negative direction.

In a case where a polyhydroxyalkanoate-based resin particle having a specific color value for which the L* value, the a* value, and the b* value in the L*a*b* color space are within the above-described ranges, respectively, has been made into a compounding agent that is to be used for topical preparations (cosmetics) such as compounding agents for slipperiness imparting agents for foundations, antiperspirants, and the like, it is easier to match the tint than normal white particles, and it is possible to make the polyhydroxyalkanoate-based resin particle less noticeable and look naturally.

### <Other Components>

The polyhydroxyalkanoate-based resin particle of the present invention may contain a flow modifier, an ultraviolet absorber, a light stabilizer, a pigment (for example, an extender pigment, a color pigment, a metal pigment, a mica powder pigment, or the like), a dye, a moisturizer, a surfactant, a resin other than the polyhydroxyalkanoate-based resin, a fragrance, a clay mineral, a preservative and disinfectant, an anti-inflammatory agent, an antioxidant, an ultraviolet absorber, a pH adjuster (triethanolamine or the like), a specially formulated additive, a pharmaceutical active ingredient, or the like as necessary.

### <Application>

The polyhydroxyalkanoate-based resin particle of the present invention can be used in applications such as compounding agents for topical preparations including a variety of cosmetics such as foundations, antiperspirants, and scrubbing agents, compounding agents for coating materials, compounding agents for resin compositions; anti-blocking agents, matting agents for coating materials (paints), a variety of agents such as rheology modifiers, slipperiness imparting agents, light diffusing agents, auxiliary agents for fine ceramics sintering and molding, fillers for adhesives, and medical diagnostic testing agents, additives for molded products such as automotive materials and building materials, and the like.

<Method for Producing Polyhydroxyalkanoate-Based Resin Particle>

A method for producing the polyhydroxyalkanoate-based resin particle of the present invention is not particularly limited. Examples of the method include
(I) a production method including a step of mixing and kneading a polyhydroxyalkanoate-based resin in a molten state and an emulsifier aqueous solution to obtain a slurry of the polyhydroxyalkanoate-based resin particle, then, performing cleaning and a dehydration treatment on the slurry, and drying and then crushing the obtained cake of the polyhydroxyalkanoate-based resin particle (production method I) and
(II) a production method including a step of heating a polyhydroxyalkanoate-based resin in the presence of: an organic solvent containing an alcoholic solvent; water; and a dispersion stabilizer, to emulsify and disperse the resin, and cooling the resin to obtain a dispersion of the polyhydroxyalkanoate-based resin, then, separating and drying a polyhydroxyalkanoate-based resin particle from the dispersion (production method II).

### <Production Method I>

Examples of the production method I pertaining to the production of the polyhydroxyalkanoate-based resin particle of the present invention include production methods including steps represented by the following (I-1) to (I-4).
(I-1) A step of performing the mixing and kneading of a polyhydroxyalkanoate-based resin in a molten state and an emulsifier aqueous solution by mixing and kneading a polyhydroxyalkanoate-based resin raw material powder or a polyhydroxyalkanoate-based resin raw material pellet and the emulsifier aqueous solution using a disperser to obtain a polyhydroxyalkanoate-based resin slurry (kneading and slurry production step)
(I-2) A step of performing cleaning and a dehydration treatment on the polyhydroxyalkanoate-based resin slurry (cleaning and dehydration treatment step)
(I-3) A step of drying, then, crushing, and classifying as necessary a cake of a polyhydroxyalkanoate-based resin particle obtained in the (I-2), thereby obtaining the polyhydroxyalkanoate-based resin particle (particle production step)

In the polyhydroxyalkanoate-based resin particle that is obtained by the steps (I-1) to (I-3), any one or more of the total remaining amount of an unsaturated fatty acid, such as crotonic acid, a hexenoic acid, or a pentenoic acid, which is a decomposition product, the total remaining amount of crotonic acid, and the total remaining amount of a pentenoic acid have been reduced. This makes it possible to suppress the odor of the polyhydroxyalkanoate-based resin particle.

### (Kneading and Slurry Production Step)

### {Emulsifier Aqueous Solution}

The emulsifier aqueous solution that is used in the kneading and slurry production step is not particularly limited as long as the solution contains at least an emulsifier and water as components and is capable of emulsifying the polyhydroxyalkanoate-based resin in a molten state and capable of forming a slurry.

The emulsifier that composes the emulsifier aqueous solution is not particularly limited. Examples thereof include one or more surfactants selected from the group consisting of anionic surfactants containing a fatty acid salt having 4 to 18 carbon atoms, such as sodium lauryl sulfate and, furthermore, sodium oleate; cationic surfactants such as lauryl trimethyl ammonium chloride; amphoteric surfactants such as N-lauryl glycine; nonionic surfactants such as nonyl phenyl polyethylene oxide, glycerin fatty acid esters, sucrose fatty acid esters, sorbitan fatty acid esters, propylene glycol fatty acid esters, lecithin, and alkylene oxide adducts thereof; and the like. In addition, examples thereof include one or more selected from the group consisting of natural polymers such as starch, casein, gelatin, alginic acid, alginates, locust bean gum, guar gum, gum arabic, xanthan gum, agar, carrageenan, crystalline cellulose, and pectin; semi-synthetic polymers such as hydroxyethyl cellulose, methyl cellulose, carboxymethyl cellulose, propylene glycol alginate, and cationic modified starch; water-soluble polymers such as polyvinyl alcoholic resins, polyacrylamide, polyvinylpyrrolidone, polyacrylic acid, polyethyleneimine, anionic or cationic modified products thereof, hydrophobic modified products thereof, poly(meth)acrylic acid, polyvinylpyrrolidone, polyvinylamine, poly(anhydride)maleic acid, polystyrene sulfonic acid, copolymers of (meth)acrylic acid or maleic anhydride and a vinyl-based monomer (for example, (meth)acrylic acid ester, an aromatic vinyl-based monomer (styrene or the like), an olefin-based monomer, or the like), modified polyesters (modified products by succinic anhydride, maleic anhydride, polyethylene oxide, or the like), and polyoxyethylene-based polymers.

In the present invention, a biodegradable emulsifier is preferable in consideration of a case where the emulsifier remains, and for example, one or more selected from the group consisting of polyvinyl alcoholic resins (polyvinyl alcohol, partially saponified polyvinyl alcohol, partially saponified polyvinyl alcohol containing a functional group (for example, a sulfonic acid group, a carboxyl group, an amino group, or the like), partially saponified polyvinyl alcohol containing a terminal hydrophobic group, and the like), starch, modified polyesters (modified products by succinic anhydride, maleic anhydride, polyethylene oxide, or the like), polyoxyethylene-based polymers are preferable. A polyvinyl alcohol-based resin is particularly preferable.

### {Disperser}

The disperser is not particularly limited as long as the disperser is capable of putting the polyhydroxyalkanoate-based resin raw material powder or the polyhydroxyalkanoate-based resin raw material pellet into a molten state and capable of applying a shear force necessary to produce the particle of the polyhydroxyalkanoate-based resin in a molten state. Examples thereof include dispersers selected from the group consisting of a variety of extruders, a homomixer, a homogenizer, a colloid mill, a kneader-ruder, a high-performance disperser, a stirrer for high-viscosity liquids, and the like. A screw extruder is preferable, and a twin screw co-rotating extruder or a single screw extruder is more preferable.

A method for mixing and kneading the polyhydroxyalkanoate-based resin raw material powder or the polyhydroxyalkanoate-based resin raw material pellet and the emulsifier aqueous solution using dispersion means is not particularly limited. Examples thereof include
(i) a method in which the emulsifier aqueous solution is added to the polyhydroxyalkanoate-based resin that has been put into a molten state under stirring,
(ii) a method in which both the polyhydroxyalkanoate-based resin raw material powder or the polyhydroxyalkanoate-based resin raw material pellet and the emulsifier aqueous solution are prepared, mixed, and kneaded at once, and the like.

In a case where the extruder is used, the method (i) is preferable.

In the method (i), in a case where a screw extruder, such as a twin screw co-rotating extruder, is used, for example, it is preferable to continuously supply the polyhydroxyalkanoate-based resin raw material powder or the polyhydroxyalkanoate-based resin raw material pellet from a hopper portion of the extruder, continuously press-fit the emulsifier aqueous solution from a supply port installed at an arbitrary position other than the point in time of melting the resin in the extruder, and mix and knead the raw material powder or the raw material pellet and the emulsifier aqueous solution. The supply ports of the emulsifier aqueous solution may be provided at a plurality of places as necessary. This makes it possible to perform the melting of the polyhydroxyalkanoate-based resin and the mixing and kneading with the emulsifier aqueous solution and makes it possible to continuously produce the polyhydroxyalkanoate-based resin slurry.

### (Cleaning and Dehydration Treatment Step)

The cleaning and dehydration treatment step includes an operation of adding water to the polyhydroxyalkanoate-based resin slurry obtained in the kneading and slurry production step and then performing a dehydration treatment thereon and can be performed once or more. The cleaning and dehydration treatment step makes the polyhydroxyalkanoate-based resin in the polyhydroxyalkanoate-based resin slurry cleaned, and the dehydration treatment makes it possible to obtain a cake of the polyhydroxyalkanoate-based resin particle. For the dehydration treatment, for example, a centrifugal dehydrator or a pressure dehydrator can be used. In the cleaning and dehydration treatment step, the amount of water added, the number of times of the cleaning and dehydration treatment performed, the dehydration treatment conditions, and the like are not particularly limited and can be appropriately set.

### (Particle Production Step)

The particle production step includes an operation of drying, then, crushing, and classifying as necessary the cake of the polyhydroxyalkanoate-based resin particle obtained in the cleaning and dehydration treatment step, which makes it possible to obtain the polyhydroxyalkanoate-based resin particle.

The drying conditions and the crushing conditions are not particularly limited and can be appropriately set.

The crushed polyhydroxyalkanoate-based resin particle may be classified as necessary. Examples of a classification method include air classification, air flow classification, screen classification, and the like. The classification is preferably performed in an air atmosphere where the relative humidity is 30% or less and preferably 20% or less in order to prevent the polyhydroxyalkanoate-based resin particle from absorbing moisture in the air.

The obtained polyhydroxyalkanoate-based resin particle is preferably sealed with a packaging material that does not easily transmit moisture and stored as packaged goods to prevent absorption of moisture in the air.

### <Production Method II>

Examples of the production method II pertaining to the production of the polyhydroxyalkanoate-based resin particle of the present invention include production methods including steps represented by the following (II-1) to (II-4).
(II-1) A step of heating and stirring a polyhydroxyalkanoate-based resin in the presence of: an organic solvent containing at least one alcoholic solvent; water; and a dispersion stabilizer, at a temperature of, for example, 110°C or higher and 180°C or lower to emulsify and disperse the resin to obtain a dispersion of the polyhydroxyalkanoate-based resin (emulsification and dispersion step)
(II-2) A step of obtaining a polyhydroxyalkanoate-based resin particle dispersion having a surface coated with the dispersion stabilizer by cooling the dispersion after the emulsification and dispersion step (cooling and particle production step)
(II-3) A step of removing the dispersion stabilizer that has coated the surface as necessary to obtain the polyhydroxyalkanoate-based resin particle dispersion from which the dispersion stabilizer on the surface has been removed (dispersion stabilizer removal step)
(II-4) A step of filtering, cleaning, dehydrating, drying, and, as necessary, classifying the polyhydroxyalkanoate-based resin particle dispersion obtained in the (II-2) or (II-3), thereby obtaining the polyhydroxyalkanoate-based resin particle (particle production step)

In the polyhydroxyalkanoate-based resin particle that is obtained by the steps (II-1) to (II-4), any one or more of the total remaining amount of an unsaturated fatty acid, such as crotonic acid, a hexenoic acid, or a pentenoic acid, which is a decomposition product, the total remaining amount of crotonic acid, and the total remaining amount of a pentenoic acid have been reduced, and furthermore, the content of the organic solvent or the like has been reduced. This makes it possible to suppress the odor of the polyhydroxyalkanoate-based resin particle.

### (Emulsification and Dispersion Step)

### {Organic Solvent Containing Alcoholic Solvent}

In the production method II of the polyhydroxyalkanoate-based resin particle of the present invention, the alcoholic solvent that is contained in the organic solvent that is used in the emulsification and dispersion step is not particularly limited. Examples thereof include one or more selected from the group consisting of methanol, ethanol, propanol, hexanol, ethylene glycol, diethylene glycol, 3-alkoxy-3-methyl-1-butanol, 3-alkoxy-3-methyl-1-butylacetate (here, the number of carbon atoms in an alkoxy group in all of the alcoholic solvents is one to five), and the like.

In the present invention, 3-alkoxy-3-methyl-1-butanol and/or 3-alkoxy-3-methyl-1-butylacetate (hereinafter, also referred to as the specific solvent) is preferably contained. The proportion of the specific solvent in the organic solvent is, for example, 10 mass% or more, preferably 20 mass% or more, and more preferably 30 mass% or more and is, for example, 90 mass% or less, preferably 80 mass% or less, and more preferably 70 mass% or less from the viewpoint of oil absorption characteristics and particle production. Examples of solvents other than the specific solvent that may be contained in the organic solvent include lower alcohols such as methanol and ethanol and acetate ester-based solvents such as ethyl acetate and butyl acetate.

As the specific solvent, a solvent that has been made commercially available by Kuraray Co., Ltd. under the trade name of SOLFIT can also be used. In addition, 3-alkoxy-3-methyl-1-butanol can be produced by a well-known method (for example, a method described in WO 2013/146370 or the like). The numbers of carbon atoms in alkoxy groups in the specific solvent are each independently one to five. In a case where the number of carbon atoms in the alkoxy group is larger than five, the solubility deteriorates in some cases. Specific examples of the alkoxy group include a methoxy group, an ethoxy group, a propoxy group, a butoxy group, and a pentyloxy group. In the propoxy group, the butoxy group, and the pentyloxy group, not only a straight chain but also an alternative structural isomer are included. A preferable alkoxy group is a methoxy group, an ethoxy group, and a propoxy group.

3-Alkoxy-3-methyl-1-butanol and 3-alkoxy-3-methyl-1-butyl acetate (here, the number of carbon atoms in an alkoxy group in all of the alcoholic solvents is one to five) are highly stable alcoholic solvents, and the use of these makes it possible to produce a polyhydroxyalkanoate-based resin particle having a spherical shape, a controlled volume average particle diameter, a narrow particle size distribution, and an excellent color tone.

In addition, 3-alkoxy-3-methyl-1-butanol and/or 3-alkoxy-3-methyl-1-butyl acetate is biodegradable and has low skin irritation, and it is thus possible to suppress the adverse influence of the remaining specific solvent when used in an application such as a cosmetic. Particularly, 3-alkoxy-3-methyl-1-butanol and/or 3-alkoxy-3-methyl-1-butyl acetate is useful as a solvent at the time of producing the particle of the polyhydroxyalkanoate-based resin in a wet manner.

Additionally, 3-alkoxy-3-methyl-1-butanol and/or 3-alkoxy-3-methyl-1-butyl acetate dissolves or plasticizes the polyhydroxyalkanoate-based resin at a high temperature of, for example, 110°C or higher, but does not dissolve the polyhydroxyalkanoate-based resin at normal temperature (25°C), and the alcoholic solvent can be thus easily reused, which is industrially advantageous. Furthermore, the use of 3-alkoxy-3-methyl-1-butanol and 3-alkoxy-3-methyl-1-butyl acetate (here, the number of carbon atoms in an alkoxy group in all of the alcoholic solvents is one to five) as the alcoholic solvent makes it possible to improve the oil absorption characteristics of the polyhydroxyalkanoate-based resin particle.

### {Dispersion Stabilizer}

In the method for producing the polyhydroxyalkanoate-based resin particle of the present invention, the dispersion stabilizer that is used in the emulsification and classification step is not particularly limited. The dispersion stabilizer simply needs to function as a dispersion stabilizer when the polyhydroxyalkanoate-based resin is emulsified and dispersed by heating and stirring, for example, heating and stirring at 110°C or higher and 180°C or lower, in the presence of at least one alcoholic solvent and water. For example, the surface of the dispersion stabilizer may be treated with a silane coupling agent, a non-animal surface treatment agent, or the like, and particularly, a poorly water-soluble inorganic compound particle is preferable as the dispersion stabilizer.

The poorly water-soluble inorganic compound is a substance having a solubility in water of less than 2.0 g/L, preferably less than 1.0 g/L, more preferably less than 100 mg/L, and still more preferably less than 50 mg/L. Examples thereof include one or more selected from the group consisting of calcium carbonate, barium carbonate, magnesium carbonate, silica, alumina, titanium oxide, calcium sulfate, barium sulfate, magnesium sulfate, tricalcium phosphate, magnesium phosphate, aluminum phosphate, zinc phosphate, calcium pyrophosphate, magnesium pyrophosphate, aluminum pyrophosphate, zinc pyrophosphate, calcium metasilicate, and the like. Among these, a carbonate, particularly, calcium carbonate is preferable from the viewpoint of ease of removal after use.

The surface treatment agent that treats the surface of the poorly water-soluble inorganic compound is not particularly limited as long as the surface treatment agent is capable of imparting hydrophobicity. Examples thereof include one or more selected from the group consisting of oils such as hydrocarbon oils, ester oils, and lanolin; silicones such as dimethylpolysiloxane, methylhydrogenpolysiloxane, and methylphenylpolysiloxane; fluorine compounds such as perfluoroalkyl group-containing esters, perfluoroalkylsilanes, perfluoropolyethers, and polymers having a perfluoroalkyl group; silane coupling agents such as 3-methacryloxypropyltrimethoxysilane and 3-glycidoxypropyltrimethoxysilane; titanium coupling agents such as isopropyl triisostearoyl titanate and isopropyl tris(dioctylpyrophosphate)titanate; metal soaps; fatty acids; amino acids such as acylglutamic acid, lecithins such as hydrogenated egg yolk lecithin, and the like.

In particular, calcium carbonate that has been surface-treated with a silane coupling agent or a non-animal surface treatment agent is highly compatible with the polyhydroxyalkanoate-based resin, has excellent dispersion stability, and makes it easy to control the particle diameter of the polyhydroxyalkanoate-based resin particle, and calcium carbonate is thus preferable.

The particle diameter of the poorly water-soluble inorganic compound particle is not particularly limited, but the primary particle diameter is preferably small to increase the specific surface area. The average primary particle diameter of the poorly water-soluble inorganic compound particle is, for example, 10 nm or more and is, for example, 1000 nm or less, preferably 500 nm or less, and more preferably 200 nm or less.

The amount of the organic solvent used in the emulsification and dispersion step is, for example, 5 parts by mass or more and is, for example, 1,200 parts by mass or less, preferably 800 parts by mass or less, and more preferably 500 parts by mass or less relative to 100 parts by mass of the polyhydroxyalkanoate-based resin particle from the viewpoint of performing sufficient stirring and mixing and the viewpoint of the productivity.

The amount of the dispersion stabilizer used in the emulsification and dispersion step is, for example, 5 mass% or more and is, for example, 50 mass% or less, preferably 40 mass% or less, and more preferably 35 mass% or less relative to the polyhydroxyalkanoate-based resin particle from the viewpoint of performing sufficient stirring and mixing and the viewpoint of the productivity. In addition, it is also possible to jointly use a variety of surfactants in addition to the dispersion stabilizer, which is the poorly water-soluble inorganic compound. As the amount of the surfactant added, for example, 0.01 to 0.5 parts by mass of the surfactant can be used relative to 100 parts by mass of water.

At the time of heating and stirring the polyhydroxyalkanoate-based resin particle, the particle can be stirred by means such as a liquid phase stirring method with a stirring blade, a mixing method with a homogenizer, or a mixing method by ultrasonic irradiation. The speed and time of the stirring are not particularly limited as long as the polyhydroxyalkanoate-based resin uniformly disperses in the solvent.

### (Cooling and Particle Production Step)

A method for cooling the polyhydroxyalkanoate-based resin dispersion is not particularly limited. In the present invention, it is preferable to slowly cool the dispersion from the heating temperature to the cooling temperature at a cooling rate of, for example, 0.5°C/minute or faster or 5.0°C/minute or slower. In addition, the cooling is preferably performed under stirring. The stirring rate can be set within the same range as that of the stirring rate during the heating and stirring. The cooling temperature is not particularly limited, but is, for example, 5°C or higher and 45°C or lower.

This makes it possible to obtain the polyhydroxyalkanoate-based resin particle dispersion having a surface coated with the dispersion stabilizer (the poorly water-soluble inorganic compound or the like).

### (Dispersion Stabilizer Removal Step and Particle Production Step)

The polyhydroxyalkanoate-based resin particle dispersion having a surface coated with the dispersion stabilizer (the poorly water-soluble inorganic compound), which has been obtained by cooling, is separated from the solvent by filtering, cleaning, dehydration, and drying and, as necessary, classified, whereby the polyhydroxyalkanoate-based resin particle of the present invention is obtained.

In the case of obtaining a polyhydroxyalkanoate-based resin particle having a surface not coated with the dispersion stabilizer (the poorly water-soluble inorganic compound), a step of removing the dispersion stabilizer using an acid or the like that decomposes and dissolves the dispersion stabilizer (the poorly water-soluble inorganic compound) before filtering may be added. At the time of decomposing and dissolving the dispersion stabilizer, it is preferable to add the acid to an extent that the acid does not become a strong acid, for example, the acid for which the necessary number of moles is 1.05 to 1.50 times and more preferably 1.05 to 1.20 times, stir the dispersion stabilizer at 40°C or lower, and filter and clean the dispersion stabilizer for 24 hours or shorter and more preferably 12 hours or shorter from the viewpoint of suppressing the hydrolysis of the polyhydroxyalkanoate-based resin and preventing the degradation of the spread of the particle on skin when added to a topical preparation.

After that, the polyhydroxyalkanoate-based resin particle dispersion is dried by a vacuum drying method or a spray drying method, whereby the polyhydroxyalkanoate-based resin particle of the present invention can be obtained.

The dried polyhydroxyalkanoate-based resin particle may be classified as necessary. Examples of a classification method include air classification, air flow classification, screen classification, and the like. The classification is preferably performed in an air atmosphere where the relative humidity is 30% or less and preferably 20% or less in order to prevent the polyhydroxyalkanoate-based resin particle from absorbing moisture in the air.

The obtained polyhydroxyalkanoate-based resin particle is preferably sealed with a packaging material that does not easily transmit moisture and stored as packaged goods to prevent absorption of moisture in the air.

### [Topical Preparation]

A topical preparation of the present invention contains the polyhydroxyalkanoate-based resin particle of the present invention. The kind of the topical preparation is not particularly limited. Examples thereof include make-up cosmetics such as powders, face powders (loose powder, pressed powder, and the like), foundations (powder foundation, liquid foundation, emulsion-type foundation, and the like), lipsticks, lip balms, blushers, eyebrow and eye cosmetics, and nail polish; cleansing cosmetics such as soap, body shampoo, face wash cream, facial scrub cleanser, and toothpaste; lotions such as pre-shave lotion and body lotion, topical body preparations such as body powder and baby powder, skin care cosmetics such as skin toners, creams, milky lotions (cosmetic milky lotions), sunscreen cosmetics, suntan products, antiperspirants (liquid antiperspirants, solid antiperspirants, cream antiperspirants, and the like), facial masks, hair washing cosmetics, hair dyes, hair styling products, aromatic cosmetics, bath preparations, shaving cream, and the like.

Particularly, cosmetics are preferable, and make-up cosmetics, cleansing cosmetics, skin care cosmetics, and the like are more preferable from the viewpoint of taking advantage of the characteristics, such as a low odor and underwater biodegradability, of the polyhydroxyalkanoate-based resin particle.

In a case where the topical preparation of the present invention is a cosmetic, the content of the polyhydroxyalkanoate-based resin particle can be adjusted as appropriate depending on the kind of the cosmetic. The content is, for example, 0.1 mass% or more, preferably 0.5 mass% or more, and more preferably 1 mass% or more, and is, for example, 50 mass% or less, preferably 30 mass% or less, and more preferably 25 mass% or less from the viewpoint of making the production cost, the stability, and the texture favorable.

A main agent or additive that is generally used can be blended into the topical preparation of the present invention to an extent that the effect of the present invention is not impaired. Examples of such a main agent and additive include one or more selected from the group consisting of water, lower alcohols (alcohols having five or less carbon atoms), fats and oils, waxes, hydrocarbons, higher fatty acids, higher alcohols, sterols, fatty acid esters, metal soaps, moisturizers, surfactants, polymer compounds, coloring material raw materials, fragrances, clay minerals, preservatives and disinfectants, anti-inflammatory agents, antioxidants, ultraviolet absorbers, organic-inorganic composite particles, pH adjusters (triethanolamine and the like), specially formulated additives, pharmaceutical active ingredients, and the like.

Examples of the fats and oils and the waxes include one or more selected from the group consisting of avocado oil, almond oil, olive oil, cocoa butter, beef tallow, sesame oil, wheat germ oil, safflower oil, shea butter, turtle oil, camellia oil, persic oil, castor oil, grape oil, macadamia nut oil, mink oil, egg yolk oil, Japan wax, coconut oil, rosehip oil, hydrogenated oil, silicone oil, orange roughy oil, carnauba wax, candelilla wax, whale wax, jojoba oil, montan wax, beeswax, lanolin, and the like.

Examples of the hydrocarbons include one or more selected from the group consisting of liquid paraffin, Vaseline, paraffin, ceresin, microcrystalline wax, squalane, and the like.

Specific examples of the higher fatty acids include one or more selected from fatty acids having 11 or more carbon atoms, such as lauric acid, myristic acid, palmitic acid, stearic acid, oleic acid, behenic acid, undecylenic acid, oxystearic acid, linoleic acid, lanolin fatty acid, and synthetic fatty acids.

Specific examples of the higher alcohols include one or more selected from the group consisting of alcohols having six or more carbon atoms, such as lauryl alcohol, cetyl alcohol, cetostearyl alcohol, stearyl alcohol, oleyl alcohol, behenyl alcohol, lanolin alcohol, hydrogenated lanolin alcohol, hexyldecanol, octyldecanol, isostearyl alcohol, jojoba alcohol, and decyltetradecanol.

Specific examples of the sterols include one or more selected from the group consisting of cholesterol, dihydrocholesterol, phytocholesterol, and the like.

Examples of the fatty acid esters include one or more selected from the group consisting of linoleic acid esters such as ethyl linoleate; lanolin fatty acid esters such as isopropyl lanolinate; lauric acid esters such as hexyl laurate; myristate esters such as isopropyl myristate, myristyl myristate, cetyl myristate, and octyldodecyl myristate; oleic acid esters such as decyl oleate and octyldodecyl oleate; dimethyloctanoic acid esters such as hexyldecyl dimethyloctanoate; isooctanoic acid esters such as cetyl isooctanoate (cetyl 2-ethylhexanoate); isononanoic acid esters such as ethylhexyl isononanoate, isononyl isononanoate, and isotridecyl isononanoate; palmitic acid esters such as isopropyl palmitate, ethylhexyl palmitate, and decyl palmitate; cyclic alcohol fatty acid esters such as glyceryl trimyristate, tri(caprylic/capric acid)glycerin, propylene glycol dioleate, glyceryl triisostearate, glyceryl triisooctanoate, cetyl lactate, myristyl lactate, diisostearyl malate, cholesteryl isostearate, and cholesteryl 12-hydroxystearate; and the like.

As the fats and oils, the waxes, the hydrocarbons, the higher fatty acids, the higher alcohols, the sterols, and the oils such as fatty acid ester oils, fixed oils are preferable, fixed oils having a viscosity at 20°C of 550 mPa·s or lower are more preferable, fixed oils having a viscosity of 1 to 550 mPa·s are still more preferable, and fixed oils having a viscosity of 5 to 550 mPa·s are particularly preferable. When such a fixed oil is combined with the polyhydroxyalkanoate-based resin particle of the present invention, the compatibility with oils is favorable, the particle can be uniformly applied, and effects of an even-toned bright finish after application, excellent adhesion to skin, easy spread on skin, and excellent temporal stability can be obtained. Examples of the fixed oils having a viscosity at 20°C of 550 mPa·s or lower include one or more selected from the group consisting of liquid paraffin, squalane, olive oil, castor oil, jojoba oil, mink oil, macadamia nut oil, hexyl laurate, isopropyl myristate, octyldodecyl myristate, cetyl isooctanoate (cetyl 2-ethylhexanoate), ethylhexyl isononanoate, isononyl isononanoate, isotridecyl isononanoate, isopropyl palmitate, ethylhexyl palmitate, decyl palmitate, caprylic/capric triglyceride, glyceryl triisostearate, glyceryl triisooctanoate, and the like. The content of the fixed oil in a cosmetic of the present invention is preferably 1 to 20 mass% from the viewpoint of exhibiting the above-described effects. The fixed oil in the present specification means an oil that remains on skin at least for several hours at room temperature (23°C) under the atmospheric pressure and particularly has a vapor pressure of lower than 0.13 Pa (0.01 mmHg).

Examples of the metal soaps include one or more selected from the group consisting of zinc laurate, zinc myristate, magnesium myristate, zinc palmitate, zinc stearate, aluminum stearate, calcium stearate, magnesium stearate, zinc undecylenate, and the like.

Examples of the moisturizers include one or more selected from the group consisting of glycerin, propylene glycol, 1,3-butylene glycol, polyethylene glycol, sodium dl-pyrrolidone carboxylate, sodium lactate, sorbitol, sodium hyaluronate, polyglycerin, xylitol, maltitol, and the like.

Examples of the surfactants include one or more selected from the group consisting of anionic surfactants such as higher fatty acid soaps, higher alcohol sulfate esters, N-acylglutamates, and phosphate ester salts; cationic surfactants such as amine salts and quaternary ammonium salts; amphoteric surfactants such as betaine type, amino acid type, imidazoline type, and lecithin; nonionic surfactants such as fatty acid monoglycerides, polyethylene glycols, propylene glycol fatty acid esters, sorbitan fatty acid esters, sucrose fatty acid esters, polyglycerin fatty acid esters, and ethylene oxide condensates; and the like.

Examples of the polymer compounds include one or more selected from the group consisting of natural polymer compounds such as gum arabic, gum tragacanth, guar gum, locust bean gum, karaya gum, iris moss, quince seed, gelatin, shellac, rosin, and casein; semi-synthetic polymer compounds such as sodium carboxymethylcellulose, hydroxyethylcellulose, methylcellulose, ethylcellulose, sodium alginate, ester gum, nitrocellulose, hydroxypropylcellulose, and crystalline cellulose; synthetic polymer compounds such as polyvinyl alcohol, polyvinylpyrrolidone, sodium polyacrylate, carboxyvinyl polymer, polyvinyl methyl ether, polyamide resin, silicone oil, and resin particles such as nylon particles, poly(meth)acrylic acid ester particles (for example, polymethyl methacrylate particles and the like), polystyrene particles, silicone particles, urethane particles, polyethylene particles, and silica particles; and the like.

Examples of the coloring material raw materials include one or more selected from the group consisting of inorganic pigments such as iron oxides (red iron oxide, yellow iron oxide, black iron oxide, and the like), ultramarine, ferric ferrocyanide, chromium oxide, chromium hydroxide, carbon black, manganese violet, titanium oxide, zinc oxide, talc, kaolin, calcium carbonate, magnesium carbonate, mica, aluminum silicate, barium silicate, calcium silicate, magnesium silicate, silica, zeolite, barium sulfate, calcined calcium sulfate (calcined gypsum), calcium phosphate, hydroxyapatite, and ceramic powder; tar coloring matters such as an azo-based coloring matter, a nitro-based coloring matter, a nitroso-based coloring matter, a xanthene-based coloring matter, a quinoline-based coloring matter, an anthraquinoline-based coloring matter, an indigo-based coloring matter, a triphenylmethane-based coloring matter, a phthalocyanine-based coloring matter, and a pyrene-based coloring matter; and the like.

A powder raw material such as the resin particle or the coloring material raw material can also be used after a surface treatment performed thereon in advance. As a method for the surface treatment, a well-known surface treatment technique can be used, and examples thereof include one or more treatment methods selected from the group consisting of an oil treatment with a hydrocarbon oil, an ester oil, lanolin, or the like; a silicone treatment with dimethylpolysiloxane, methylhydrogenpolysiloxane, methylphenylpolysiloxane, or the like; a fluorine compound treatment with a perfluoroalkyl group-containing ester, perfluoroalkylsilane, perfluoropolyether, a polymer having a perfluoroalkyl group, or the like; a silane coupling agent treatment with 3-methacryloxypropyltrimethoxysilane, 3-glycidoxypropyltrimethoxysilane, or the like; a titanium coupling agent treatment with isopropyl triisostearoyl titanate, isopropyl tris(dioctylpyrophosphate) titanate, or the like; a metal soap treatment; an amino acid treatment with acylglutamic acid or the like; a lecithin treatment with hydrogenated egg yolk lecithin or the like; a collagen treatment; a polyethylene treatment; a moisturizing treatment; an inorganic compound treatment; a mechanochemical treatment; and the like.

Examples of the fragrances include one or more selected from the group consisting of anisaldehyde, benzyl acetate, geraniol, and the like.

Examples of the clay minerals include components having several functions, such as extender pigments and adsorbents, for example, one or more selected from the group consisting of talc, mica, sericite, titanium sericite (sericite coated with titanium dioxide), muscovite, VEEGUM (registered trademark) manufactured by Vanderbilt Minerals, LLC, and the like.

Examples of the preservatives and disinfectants include one or more selected from the group consisting of methylparaben, ethylparaben, propylparaben, benzalkonium, benzethonium, and the like.

Examples of the antioxidants include one or more selected from the group consisting of dibutylhydroxytoluene, butylhydroxyanisole, propyl gallate, tocopherol, and the like.

Examples of the ultraviolet absorbers include one or more selected from the group consisting of inorganic absorbers such as fine particle titanium oxide, fine particle zinc oxide, fine particle cerium oxide, fine particle iron oxide, and fine particle zirconium oxide; organic absorbers such as benzoic acid-based absorbers, para-aminobenzoic acid-based absorbers, anthranilic acid-based absorbers, salicylic acid-based absorbers, cinnamic acid-based absorbers, benzophenone-based absorbers, and dibenzoylmethane-based absorbers; and the like.

Examples of the specially formulated additives include one or more selected from the group consisting of hormones such as estradiol, estrone, ethinyl estradiol, cortisone, hydrocortisone, prednisone; vitamins such as vitamin A, vitamin B, vitamin C, and vitamin E; skin astringents such as citric acid, tartaric acid, lactic acid, aluminum chloride, aluminum potassium sulfate, aluminium chlorohydroxy allantoinate, zinc paraphenolsulfonate, and zinc sulfate; hair growth promoters such as cantharides tincture, capsicum tincture, ginger tincture, Swertia japonica extract, garlic extract, hinokitiol, carpronium chloride, glyceryl pentadecanoate, vitamin E, estrogen, and photosensitizers; whitening agents such as magnesium L-ascorbic acid phosphate and kojic acid; and the like.

### [Coating Material]

A coating material of the present invention contains the polyhydroxyalkanoate-based resin particle of the present invention. The coating material of the present invention may contain, aside from the polyhydroxyalkanoate-based resin particle of the present invention, a binder resin, an ultraviolet-curable resin, a solvent, or the like as necessary. As the binder resin, for example, resins that are soluble in organic solvents or water and emulsion-type resins that can be dispersed in water can be used.

In the present invention, the binder resin is not particularly limited. Examples thereof include one or more selected from the group consisting of biodegradable resins such as polyhydroxyalkanoate-based resins (for example, polylactic acid, polyglycolic acid, polybutylene succinate, polybutylene succinate adipate, polybutylene adipate terephthalate, poly(ethylene succinate terephthalate), poly(butylene succinate terephthalate), poly(butylene adipate terephthalate), poly(3-hydroxybutyrate), poly(3-hydroxyvalerate), poly(3-hydroxycaproate), poly(3-hydroxyheptanoate), poly(3-hydroxyoctanoate), poly(3-hydroxybutyrate 3-hydroxyhexanoate), poly(3-hydroxybutyrate-3-hydroxyvalerate), and the like), poly(ε-caprolactone), poly(β-propiolactone), polyamide 4, starch-based resins, cellulose-based resins, and glucosamine-based resins; acrylic resins, alkyd-based resins, polyamide-based resins, polyester-based resins, polyurethane-based resins, chlorinated polyolefin-based resins, amorphous polyolefin-based resins, and the like.

In the present invention, examples of the ultraviolet-curable resin include multifunctional (meth)acrylate resins such as polyhydric alcohol multifunctional (meth)acrylates; multifunctional urethane acrylate resins that are synthesized from diisocyanates, polyhydric alcohols, and (meth)acrylic acid esters having a hydroxy group; and the like. Among these, multifunctional (meth)acrylate resins are preferable, and polyhydric alcohol multifunctional (meth)acrylate resins having three or more (meth)acryloyl groups in one molecule are more preferable. Specific examples of the polyhydric alcohol multifunctional (meth)acrylate resins having three or more (meth)acryloyl groups in one molecule include one or more selected from the group consisting of trimethylolpropane tri(meth)acrylate, trimethylolethane tri(meth)acrylate, 1,2,4-cyclohexane tetra(meth)acrylate, pentaglycerol triacrylate, pentaerythritol tetra(meth)acrylate, pentaerythritol tri(meth)acrylate, dipentaerythritol triacrylate, dipentaerythritol pentaacrylate, dipentaerythritol tetra(meth)acrylate, dipentaerythritol hexa(meth)acrylate, tripentaerythritol triacrylate, tripentaerythritol hexaacrylate, and the like.

In the case of using the ultraviolet-curable resin, it is preferable to jointly use a photopolymerization initiator. The photopolymerization initiator is not particularly limited. Examples thereof include one or more selected from the group consisting of acetophenone-based polymerization initiators, benzoin-based polymerization initiators, benzophenone-based polymerization initiators, phosphine oxide-based polymerization initiators, ketal-based polymerization initiators, α-hydroxyalkylphenone-based polymerization initiators, α-aminoalkylphenone-based polymerization initiators, anthraquinone-based polymerization initiators, thioxanthone-based polymerization initiators, azo-based compounds, peroxide-based polymerization initiators (described in Japanese Patent Application Publication No. 2001-139663 or the like), 2,3-dialkyldione compound-based polymerization initiators, disulfide compound-based polymerization initiators, fluoroamine compound-based polymerization initiators, aromatic sulfonium-based polymerization initiators, onium salt-based polymerization initiators, borate salt-based polymerization initiators, active halogen compound-based polymerization initiators, and α-acyl oxime ester-based polymerization initiators.

These binder resins or ultraviolet-curable resins can be selected as appropriate depending on the adhesion of the coating material to base materials to be coated, the environment where the resins are used, or the like.

The content of each of the above-described components in the present invention also varies with the film thickness of a coated film to be formed, the average particle diameter of a resin particle group, and the coating method. The amount of a resin particle group of the present invention added is preferably 1 to 50 mass%, more preferably 3 to 45 mass%, and still more preferably 5 to 40 mass% in a case where the total content of the binder resin (the solid content in the case of using an emulsion-type aqueous resin) and the resin particle group of the present invention is set to 100 mass%.

In the present invention, the solvent is not particularly limited, but a solvent capable of dissolving or dispersing the binder resin or the ultraviolet-curable resin is preferable. Examples thereof include, in the case of an oil-based coating material (oil-based paint), hydrocarbon-based solvents such as toluene and xylene; ketone-based solvents such as methyl ethyl ketone and methyl isobutyl ketone; ester-based solvents such as ethyl acetate and butyl acetate; ether-based solvents such as dioxane, ethylene glycol diethyl ether, and ethylene glycol monobutyl ether; and the like. In the case of a water-based coating material (water-based paint), water, alcohols, and the like can be used. These solvents may be used singly, or two or more thereof may be mixed and used. The content of the solvent in the coating material is, normally, approximately 20 to 60 mass% relative to the total amount of the coating material.

One or more of these solvents can also be used as a diluent as necessary in order to adjust the viscosity of the coating material.

In the present invention, the coating material may contain, as necessary, a coated surface adjuster, a flow modifier, an ultraviolet absorber, a light stabilizer, a curing catalyst, an extender pigment, a color pigment, a metal pigment, a mica powder pigment, a dye, or the like, which is well known.

In the present invention, a method for forming a coated film using the coating material is not particularly limited, and any of well-known methods can be used. Examples thereof include methods such as a spray coating method, a roll coating method, a brush coating method, an inkjet method, a bar coating method, and an immersion method, and in the case of coating a base material such as a film as a thin layer, examples thereof include a coating reverse roll coating method, a gravure coating method, a die coating method, and a comma coating method.

In addition, a crosslinked coated film can be formed by coating an arbitrary coating surface of a base material or the like with the coating material to produce a coating film, drying this coating film, and then curing the coating film as necessary.

The coating material is used to form coated films by coating a variety of base materials with the coating material. The base materials are not particularly limited, and examples thereof include metals, wood, glass, ceramics, plastics, and the like. In the present invention, it is also possible to coat a transparent base material such as polyethylene terephthalate (PET), polycarbonate (PC), or acrylic with the coating material and use the transparent base material.

### [Resin Composition]

A resin composition of the present invention contains the polyhydroxyalkanoate-based resin particle of the present invention. The resin composition of the present invention may contain, aside from the polyhydroxyalkanoate-based resin particle of the present invention, a base material resin.

In the present invention, the base material resin is not particularly limited. Examples thereof include one or more selected from the group consisting of biodegradable resins such as polyhydroxyalkanoate-based resins (for example, polylactic acid, polyglycolic acid, polybutylene succinate, polybutylene succinate adipate, polybutylene adipate terephthalate, poly(ethylene succinate terephthalate), poly(butylene succinate terephthalate), poly(butylene adipate terephthalate), poly(3-hydroxybutyrate), poly(3-hydroxyvalerate), poly(3-hydroxycaproate), poly(3-hydroxyheptanoate), poly(3-hydroxyoctanoate), poly(3-hydroxybutyrate 3-hydroxyhexanoate), poly(3-hydroxybutyrate 3-hydroxyvalerate), and the like), poly(ε-caprolactone), poly(β-propiolactone), polyamide 4, starch-based resins, cellulose-based resins, and glucosamine-based resins; thermoplastic resins such as polycarbonate, polyethylene terephthalate, polybutylene terephthalate, polyamide 6, polyamide 66, polyamide 12, an ABS resin (acrylonitrile-butadiene-styrene copolymer resin), an AS resin (acrylonitrile-styrene copolymer resin), polyethylene, polypropylene, polyacetal, polyamideimide, polyethersulfone, polyimide, polyphenylene oxide, polyphenylene sulfide, polystyrene, a thermoplastic polyurethane elastomer, a thermoplastic polyester elastomer, a thermoplastic polyamide elastomer, polyvinyl chloride, polyvinylidene fluoride, an ethylene tetrafluoroethylene copolymer (ETFE resin), a tetrafluoroethylene perfluoroalkyl vinyl ether copolymer (PFA resin), and polyether ketone; thermosetting resins such as epoxy resins and urethane-based resins; and the like.

In the present invention, the content of the polyhydroxyalkanoate-based resin particle of the present invention in the resin composition also varies with the thickness of a molded product to be formed, the average particle diameter of the polyhydroxyalkanoate-based resin particle, and the molding method. The content of the polyhydroxyalkanoate-based resin particle of the present invention added is preferably 0.1 to 70 mass%, more preferably 0.5 to 50 mass%, and still more preferably 1 to 30 mass% in a case where the total content of the base material resin and the polyhydroxyalkanoate-based resin particle of the present invention is set to 100 mass%.

In the present invention, the resin composition may contain a well-known additive as necessary. Examples of the additive include reinforced fibers such as glass fibers and carbon fibers, a flame retardant, a flow modifier, an ultraviolet absorber, a heat stabilizer, a light stabilizer, a lubricant, an extender pigment, a color pigment, a metal pigment, a dye, and the like.

In the present invention, a method for producing the resin composition is not particularly limited, and the resin composition can be produced by mixing the resin particle group and the base material resin by a well-known conventional method such as a mechanical grinding and mixing method. In the mechanical grinding and mixing method, the resin composition can be produced by, for example, mixing and stirring the resin particle group and the base material resin using a device such as a Henschel mixer, a V-type mixer, a turbula mixer, a hybridizer, or a rocking mixer.

In the present invention, a method for forming a molded product using the resin composition is not particularly limited, and any well-known methods can also be used. A molded product having an arbitrary shape suitable for an automotive material, a building material, a packaging material, or the like can be obtained by, for example, mixing the resin particle group of the present invention and the base material resin with a mixer, kneading the resin particle group and the base material resin with a melt kneader such as an extruder to obtain a pellet composed of the resin composition, and then molding this pellet by extrusion, injection, blowing, or the like.

### [Anti-blocking Agent]

An anti-blocking agent of the present invention contains the polyhydroxyalkanoate-based resin particle of the present invention. The anti-blocking agent may contain, aside from the polyhydroxyalkanoate-based resin particle of the present invention, an antioxidant, a flow modifier, a light stabilizer, a color pigment, or the like, which is well known, as necessary.

The content of the polyhydroxyalkanoate-based resin particle of the present invention in the anti-blocking agent of the present invention is preferably 70 to 100 mass%, more preferably 80 to 100 mass%, and still more preferably 90 to 100 mass%.

The anti-blocking agent of the present invention is used to impart unevenness to the surface of a resin film to prevent the surfaces of the resin films in contact with each other from adhering together and being not peeled off from each other (blocking) when the resin films have been wound together.

In the present invention, the resin film for which the anti-blocking agent can be used is not particularly limited. Examples thereof include films of one or more resins selected from the group consisting of biodegradable resins such as polylactic acid, polyglycolic acid, polybutylene succinate, polybutylene succinate adipate, polybutylene adipate terephthalate, poly(ethylene succinate terephthalate), poly(butylene succinate terephthalate), poly(butylene adipate terephthalate), poly(ε-caprolactone), poly(β-propiolactone), polyamide 4, poly(3-hydroxybutyrate), poly(3-hydroxyvalerate), poly(3-hydroxycaproate), poly(3-hydroxyheptanoate), poly(3-hydroxyoctanoate), poly(3-hydroxybutyrate/3-hydroxyhexanoate), poly(3-hydroxybutyrate/3-hydroxyvalerate), starch-based resins, cellulose-based resins, and glucosamine-based resins; polyester-based resins such as polyethylene terephthalate and polyethylene naphthalate; polyolefin-based resins such as polyethylene-based resins and polypropylene-based resins; (meth)acrylic resins; polystyrene-based resins; polyethersulfone-based resins; polyurethane-based resins; polycarbonate-based resins; polysulfone-based resins; polyether-based resins; polymethylpentene-based resins; polyetherketone-based resins; (meth)acrylonitrile-based resins; norbornene-based resins; amorphous polyolefin-based resin; polyamide resins; polyimide resins; triacetylcellulose resins; and the like.

The content of the resin particle group of the present invention in the resin film also varies with the thickness of a film to be formed, the average particle diameter of the resin particle group, and the molding method. The content of the resin particle group of the present invention is preferably 0.01 to 10 mass%, more preferably 0.01 to 5 mass%, still more preferably 0.01 to 3 mass%, and particularly preferably 0.01 to 1 mass% in the resin film.

### [Examples]

The present invention will be described below with Examples and Comparative Examples, but the present invention is not limited thereto. Unless particularly otherwise specified, "%" means "mass%."

### [Measurement Methods and the Like]

### <Remaining Amount of Unsaturated Fatty Acid>

A quantitative analysis of the remaining amount of an unsaturated fatty acid was performed as described below.

### (1) Extraction Pretreatment Method

Approximately 1 g of a polyhydroxyalkanoate-based resin particle was precisely weighed in a centrifuge tube, 5 mL of methanol was added to and mixed with the polyhydroxyalkanoate-based resin particle, ultrasonic extraction was performed for 15 minutes, and the polyhydroxyalkanoate-based resin particle and the methanol were well mixed together again. Centrifugation was performed at a rotation speed of 3,500 rpm for 30 minutes, the supernatant liquid was filtered with a 0.2 µm CHROMATODISC 13N for hydrophobic samples (manufactured by GL Sciences Inc.) to produce a test liquid, and UHPLC measurement was performed on the test liquid.

### (2) Measurement Method

The conditions for the UHPLC measurement of the test liquid were set as described below. The quantity of an unsaturated fatty acid was determined using a standard peak area value obtained by chromatography using a standard liquid. For the quantity determination, a calibration curve created with Shimadzu chromatography software LabSolutions was used. The concentration of each unsaturated fatty acid in the test liquid was obtained from this calibration curve, and the content of each unsaturated fatty acid (the remaining amount of the unsaturated fatty acid) was calculated from the obtained result.

Amount of unsaturated fatty acid (mg/kg) = measurement value (µg/mL) × amount of methanol extracted (mL) / mass of specimen (g)

### (3) UHPLC Measurement Conditions

### · Amount of Unsaturated Fatty Acid

### <UHPLC>

Device: An "NexeraX2" ultra high performance liquid chromatograph column manufactured by Shimadzu Corporation: Kinetex 1.7 µm C18 100A (2.1 mmI.D. × 50 mmL)
Column temperature: 40°C
Pump temperature: Room temperature (23°C)
Mobile phase: (A: 0.05% trifluoroacetic acid (TFA)/B: acetonitrile)
Mobile phase condition = (0 → 0.5 min = B_{conc.} 90%, 0.5 → 0.51 min = B_{conc.} 90% → 80%, 0.51 → 2 min = B_{conc.} 80%, 2 → 2.5 min = B_{conc.} 80% → 20%, 2.5 → 3 min = B_{conc.} 20%, 3 → 3.5 min = B_{conc.} 20% → 90%, 3.5 → 5 min = B_{conc.} 90%)
Flow rate: 0.6 mL/min
Measurement time: 5 min
Injection amount: 1 µL
Detector: PDA = 210 nm (crotonic acid and 2-pentenoic acid), 200 nm (4-pentenoic acid)

### · Method for Producing Standard Liquid

With the automatic dilution function of an auto sampler of a "NexeraX2" ultra high performance liquid chromatograph manufactured by Shimadzu Corporation, a 1,000 mg/L standard liquid was diluted 10 times with methanol to produce a 100 mg/L standard liquid and was diluted 20 times with methanol to produce a 50 mg/L standard liquid, respectively. Furthermore, the 100 mg/L standard liquid was diluted five times to produce a 20 mg/L standard liquid. The 50 mg/L standard liquid was diluted five times to produce a 10 mg/L standard liquid. The 20 mg/L standard liquid was diluted five times to produce a 4 mg/L standard liquid. The 10 mg/L standard liquid was diluted 10 times to produce a 1 mg/L standard liquid. The 1 mg/L standard liquid was diluted five times to produce a 0.2 mg/L standard liquid.

4-Pentenoic acid was also measured by the following method.

### · Amount of 4-Pentenoic Acid

### (1) Extraction Pretreatment Method

Approximately 1 g of a polyhydroxyalkanoate-based resin particle was weighed and immersed in 10 mL of acetone, and ultrasonic extraction was performed for 30 minutes. One milliliter of an extracted liquid (supernatant) was collected in a measurement vial, 0.5 mL of bis(trimethylsilyl)trifluoroacetamide (BSTFA) was added thereto, the extracted liquid and the bis(trimethylsilyl)trifluoroacetamide were lightly shaken to be mixed and left to stand for one hour or longer to produce a derivative (trimethylsilyl), and then the derivative was measured by gas chromatography-mass spectrometry (GC-MS).

### (2) Measurement Method

The conditions for the GC-MS measurement of the test liquid were set as described below. The quantity of an unsaturated fatty acid was determined using a standard peak area value obtained by chromatography using the standard liquid. For the quantity determination, a calibration curve created with the standard liquid was used. The concentration of each unsaturated fatty acid in the test liquid was obtained from this calibration curve, and the content of each unsaturated fatty acid (the remaining amount of the unsaturated fatty acid) was calculated from the obtained result. Amount of 4-pentenoic acid (µg/g) = concentration of 4-pentenoic acid in test liquid (µg/mL) × extracted amount (mL) / mass of specimen (g)

### (3) GC-MS Measurement Conditions

Device: Agilent Technologies 7890A GC/5975C MSD system
Column: Agilent J&W DB-5ms, 30 m × 0.25 mmi.d.
Film thickness: 0.25 µm
Inlet temperature: 250°C
Carrier gas: 1 mL/min of helium
Split ratio: 10:1
Oven temperature: 50°C (1 min) - 10°C/min - 300°C (5 min)
Injection amount: 1 µL
Ionization method: Electron impact ionization method (EI method: 70 eV)
Measurement mode: Selected ion monitoring (SIM) mode
Monitor ion: Quantitative ion m/z157 (4-pentenoic acid, TMS derivative)

### · Method for Producing Standard Liquid

0.1 g of 4-pentenoic acid was put into a 10 mL volumetric flask, and the volume was fixed with acetone, thereby preparing a 1,000 µg/mL standard raw liquid. This was diluted with acetone to prepare 500, 100, 50, 10, 5, 1, and 0.5 µg/mL standard liquids. A derivative was produced in the same manner as a specimen extracted liquid.

### <Volume Average Particle Diameter>

The volume average particle diameter of the polyhydroxyalkanoate-based resin particle was measured with a particle size distribution measuring instrument (manufactured by Beckman Coulter Inc., "Multisizer 4e"). The measurement was performed after an aperture calibrated according to the user's manual was appropriately selected depending on the size of the particle being measured.

As a specimen for measurement, a dispersion obtained by dispersing 0.1 g of the polyhydroxyalkanoate-based resin particle in 10 mL of a 0.1 mass% nonionic surfactant aqueous solution using a touch mixer (manufactured by Yamato Scientific Co., Ltd., "TOUCH MIXER MT-31") and an ultrasonic cleaner (manufactured by Velvo-Clear, "ULTRASONIC CLEANER VS-150") was used.

During the measurement, the inside of a beaker was loosely stirred so that no air bubbles entered, 100,000 polyhydroxyalkanoate-based resin particles were measured, and the particle diameter of each of the polyhydroxyalkanoate-based resin particles was obtained.

The volume average particle diameter of the polyhydroxyalkanoate-based resin particle is the arithmetic average in the volume based particle size distribution of the 100,000 particles.

### <Moisture Content>

The moisture content of the polyhydroxyalkanoate-based resin particle was measured by the Karl Fischer method as described below. That is, 0.4 g of the specimen of the polyalkanoate-based resin particle was set and measured in a "CA-200" Karl Fischer moisture meter and a "VA-236S" moisture evaporator manufactured by Mitsubishi Chemical Analytech Co., Ltd. As the anolyte and the catholyte during the measurement, AQUAMICRON AX and AQUAMICRON CXU manufactured by Mitsubishi Chemical Corporation were used, respectively. The measurement temperature was set to 150°C. As the carrier gas, N₂ was used. The flow rate of the carrier gas was set to 250 mL/min. The number of times of testing on the specimen was set to three. The moisture amount in the air alone at a specimen collection place was measured twice, and the average value thereof was regarded as the blank value. The blank value was subtracted from each measurement result, and the subtraction result was divided by the mass of the specimen, thereby obtaining the moisture content of the specimen. The moisture content of the specimen was calculated by the following equation. Moisture content = [actually measured amount of moisture (µg) - blank amount of moisture (µg)] / 1,000,000 / mass of specimen (g) × 100

Three measurement results measured by the above-described measurement method were averaged to obtain the moisture content of the specimen.

### <Average Circularity>

The average circularity of the polyhydroxyalkanoate-based resin particle was measured using a flow-type particle image analyzer (manufactured by Sysmex Corporation, "FPIA (registered trademark)-3000S").

The measurement was performed as described below.

0.05 g of sodium alkylbenzene sulfonate was added as a dispersant to 20 mL of ion exchange water to obtain a surfactant aqueous solution. 0.02 g of the polyhydroxyalkanoate-based resin particle was added to the obtained surfactant aqueous solution, and a dispersion treatment was performed by dispersing the polyhydroxyalkanoate-based resin particle in the surfactant aqueous solution using the ultrasonic cleaner (manufactured by Velvo-Clear, "VS-150") as a disperser over two minutes, thereby obtaining a dispersion liquid for measurement.

As a sheath liquid that was used in the flow-type particle image analyzer, a particle sheath (manufactured by Sysmex Corporation, "PSE-900A") was used, the dispersion liquid for measurement was introduced into the flow-type particle image analyzer equipped with a standard object lens (10 times), and the average circularity was measured under the following measurement conditions. Measurement mode: LPF measurement mode
Measurement range of particle diameter: 0.5 to 200 µm
Measurement range of particle circularity: 0.2 to 1.0
Number of particles measured: 100,000 particles

Upon the measurement, the autofocus adjustment of the flow-type particle image analyzer was performed using a suspension of a standard polymer particle (manufactured by Thermo Fisher Scientific, "5200A" (standard polystyrene particle diluted with ion exchange water)) before the beginning of the measurement. The average circularity is the average value of values obtained by dividing a perimeter calculated from the diameter of a perfect circle having the same area as the projected area of the polyhydroxyalkanoate-based resin particle in an image obtained by capturing the dispersion liquid for measurement by the perimeter of the polyhydroxyalkanoate-based resin particle in the image obtained by capturing the dispersion liquid for measurement.

### <Color Tone>

The color tone of the polyhydroxyalkanoate-based resin particle was obtained by measuring the chromaticity in the L*a*b* color space according to JIS Z 8729.

The polyhydroxyalkanoate-based resin particle was loaded up into a measurement container (powder cell) (manufactured by Konica Minolta, Inc. "CR-A50"). The L* value, a* value, and b* value of the loaded polyhydroxyalkanoate-based resin particle were measured with a color difference meter (manufactured by Konica Minolta, Inc. "CR-300").

### [Production of Resin Pellet]

### <Production Example 1>

A poly(3-hydroxybutyrate-co-3-hydroxyvalerate) resin raw material powder (manufactured by TianAn Biologic Materials Co., Ltd., "ENMAT Y1000") was continuously supplied to a 26 mm twin screw extruder ("TEM-26" manufactured by Toshiba Machine Co., Ltd.) at 15 kg/h and extracted from a strand mold having five holes with Φ5 attached to the tip of the extruder, a strand was cooled and pelletized with a pelletizer, thereby obtaining a poly(3-hydroxybutyrate-co-3-hydroxyvalerate) resin raw material pellet. The resin temperature at the mold outlet was 188°C.

### [Production of Calcium Carbonate Dispersion Liquid]

### <Production Example 2>

3,500 g of 5 mm zirconia beads, 100 g of calcium carbonate (manufactured by Shiraishi Calcium Kaisha, Ltd., "HAKUENKA PZ"; primary particle diameter: 80 nm), 450 g of ion exchange water, and 450 g of 3-methoxy-3-methyl-1-butanol ("SOLFIT FINE GRADE" manufactured by Kuraray Co., Ltd.) were added to a 2 L quartz glass pot mill and treated for 24 hours at a circumferential speed of 100 rpm on the ball mill rotating table, thereby obtaining a calcium carbonate dispersion liquid.

### [Examples and Comparative Examples]

### <Example 1>

The poly(3-hydroxybutyrate-co-3-hydroxyvalerate) resin raw material pellet obtained in Production Example 1 was continuously supplied at 7.5 kg/h to a 57 mm twin screw extruder (manufactured by Osaka Seiki, L/D = 31.6), and a partially saponified polyvinyl alcohol ("GOHSENOL GL-05" manufactured by Mitsubishi Chemical Corporation) 15% aqueous solution was continuously injected at 10.7 kg/h from the inlet provided in a cylinder portion of the third block from the input portion. A slurry containing a poly(3-hydroxybutyrate-co-3-hydroxyvalerate) resin particle dispersed in the partially saponified polyvinyl alcohol aqueous solution was continuously obtained from a rod mold having Φ7 attached to the tip of the extruder. The cylinder temperatures in the extruder were, among a total of nine blocks, 100°C in the raw material pellet input portion, 200°C in a resin melting zone 2 block, 220°C in an emulsification zone 2 block including a polyvinyl alcohol aqueous solution injection portion, 200°C in a rear portion emulsification zone 2 block, 120°C in a cooling zone 3 block, and 170°C in a tip mold.

The obtained slurry was cleaned with water (60°C) and then dehydrated using a small centrifugal dehydrator, the obtained cake of the poly(3-hydroxybutyrate-co-3-hydroxyvalerate) resin particle was dried in an oven at 80°C for 24 hours, crushed with a mixer, and furthermore, classified using a 25 µm mesh to remove coarse particles, thereby obtaining the poly(3-hydroxybutyrate-co-3-hydroxyvalerate) resin particles.

From the obtained poly(3-hydroxybutyrate-co-3-hydroxyvalerate) resin particles, none of remaining crotonic acid, remaining 2-pentenoic acid, and remaining 4-pentenoic acid was detected, and it was confirmed that the total remaining amount of the unsaturated fatty acids was 10 mass ppm or less. In addition, the volume average particle diameter was 11.2 µm, the moisture content was 0.28 mass%, the average circularity was 0.91, the L* value was 93.0, the a* value was -0.7, and the b* value was +4.3.

### <Example 2>

104 g of a poly(3-hydroxybutyrate-co-3-hydroxyvalerate) resin raw material powder (manufactured by TianAn Biologic Materials Co., Ltd., "ENMAT Y1000"), 432.2 g of 3-methoxy-3-methyl-1-butanol as a solvent, 432.2 g of ion exchange water, 31.2 g of triacetin (manufactured by Daihachi Chemical Industry Co., Ltd.), and 156 g of the calcium carbonate dispersion liquid obtained in Production Example 2 as a dispersion stabilizer were each input into a 2 L autoclave equipped with a stirring blade and a thermometer, the components were heated so that the internal temperature reached 150°C while being stirred at a rotation speed of 600 rpm, and emulsified for 90 minutes after the internal temperature reached 150°C. After that, the components were cooled to 30°C over one hour while the stirring rotation speed was maintained at 600 rpm, and a suspension was obtained.

76 mL (1.14 times the necessary number of moles) of 20% hydrochloric acid was added to the obtained suspension, the suspension was stirred for 10 minutes to decompose calcium carbonate, and poly(3-hydroxybutyrate-co-3-hydroxyvalerate) resin particles were separated using a centrifuge (manufactured by Tanabe Willtec Inc.).

The separated poly(3-hydroxybutyrate-co-3-hydroxyvalerate) resin particles were cleaned using ion exchange water that was as much as 20 times the amount of the resin added.

The cleaned poly(3-hydroxybutyrate-co-3-hydroxyvalerate) resin particles were dried over 20 hours under conditions of 60°C and a degree of vacuum of 0.05 MPa.

The dried poly(3-hydroxybutyrate-co-3-hydroxyvalerate) resin particles were classified using a classifier in which a screen having a sieve mesh size of 45 µm (manufactured by Toyo Hitec Co., Ltd., "Hi-BOLTER NR300") was installed under an air atmosphere having a relative humidity of 20%. The classification was performed by causing the poly(3-hydroxybutyrate-co-3-hydroxyvalerate) resin particles to flow in an air flow having a relative humidity of 20%, causing the air flow to collide with the screen, and removing particles having particle diameters that were too large to pass through the screen mesh.

From the obtained poly(3-hydroxybutyrate-co-3-hydroxyvalerate) resin particles, none of remaining crotonic acid, remaining 2-pentenoic acid, and remaining 4-pentenoic acid was detected, and it was confirmed that the total remaining amount of the unsaturated fatty acids was 10 mass ppm or less. In addition, the volume average particle diameter was 18.9 µm, the moisture content was 0.38 mass%, the average circularity was 0.87, the L* value was 96.3, the a* value was -0.8, and the b* value was +2.0.

### <Example 3>

The poly(3-hydroxybutyrate-co-3-hydroxyvalerate) resin raw material pellet obtained in Production Example 1 was continuously supplied at 7.5 kg/h to a 57 mm twin screw extruder (manufactured by Osaka Seiki, L/D = 31.6), and a partially saponified polyvinyl alcohol ("GOHSENOL GH-20R" manufactured by Mitsubishi Chemical Corporation) 7% aqueous solution was continuously injected at 10.7 kg/h from the inlet provided in a cylinder portion of the third block from the input portion. A slurry containing poly(3-hydroxybutyrate-co-3-hydroxyvalerate) resin particles dispersed in the partially saponified polyvinyl alcohol aqueous solution was continuously obtained from a rod mold having Φ30 attached to the tip of the extruder. The cylinder temperatures in the extruder were, among a total of nine blocks, 100°C in the raw material pellet input portion, 220°C in a resin melting zone 2 block, 220°C in an emulsification zone 2 block including a polyvinyl alcohol aqueous solution injection portion, 200°C in a rear portion emulsification zone 2 block, 160°C in a cooling zone 3 block, and 160°C in a tip mold.

The poly(3-hydroxybutyrate-co-3-hydroxyvalerate) resin particles were separated from the obtained slurry using a centrifuge (manufactured by Tanabe Willtec Inc.).

The separated poly(3-hydroxybutyrate-co-3-hydroxyvalerate) resin particles were cleaned using ion exchange water (60°C) that was as much as 20 times the amount of the resin added.

The cleaned poly(3-hydroxybutyrate-co-3-hydroxyvalerate) resin particles were dried over 20 hours under conditions of 80°C and a degree of vacuum of 0.05 MPa.

The dried poly(3-hydroxybutyrate-co-3-hydroxyvalerate) resin particles were crushed with a chopper mill and then classified using a classifier in which a screen having a sieve mesh size of 45 µm (manufactured by Toyo Hitec Co., Ltd., "Hi-BOLTER NR300") was installed under an air atmosphere having a relative humidity of 20%. The classification was performed by causing the poly(3-hydroxybutyrate-co-3-hydroxyvalerate) resin particles to flow in an air flow having a relative humidity of 20%, causing the air flow to collide with the screen, and removing particles having particle diameters that were too large to pass through the screen mesh.

From the obtained poly(3-hydroxybutyrate-co-3-hydroxyvalerate) resin particles, none of remaining crotonic acid, remaining 2-pentenoic acid, and remaining 4-pentenoic acid was detected, and it was confirmed that the total remaining amount of the unsaturated fatty acids was 10 mass ppm or less. In addition, the volume average particle diameter was 6.8 µm, the moisture content was 0.43 mass%, the average circularity was 0.92, the L* value was 92.7, the a* value was -0.4, and the b* value was +3.4.

### <Example 4>

Resin particles were obtained in the same manner as in Example 2 except that, in Example 2, the amount of triacetin was changed to 20.8 g. From the obtained poly(3-hydroxybutyrate-co-3-hydroxyvalerate) resin particles, none of remaining crotonic acid, remaining 2-pentenoic acid, and remaining 4-pentenoic acid was detected, and it was confirmed that the total remaining amount of the unsaturated fatty acids was 10 mass ppm or less. In addition, the volume average particle diameter was 32.2 µm, the moisture content was 0.31 mass%, the average circularity was 0.89, the L* value was 94.9, the a* value was -0.7, and the b* value was +2.9.

### [Preparation of Topical Preparation]

15 parts by mass of the poly(3-hydroxybutyrate-co-3-hydroxyvalerate) resin particles of Example 2, 21 parts by mass of sericite, 51 parts by mass of muscovite, 0.6 parts by mass of red iron oxide, 1 part by mass of yellow iron oxide, and 0.1 parts by mass of black iron oxide were mixed together using a Henschell mixer to produce a mixture.

Incidentally, 1 part by mass of sorbitan sesquioleate and 0.2 parts by mass of a preservative were mixed and dissolved in 10 parts by mass of cetyl 2-ethylhexanoate to produce a dissolved matter.

The mixture and the dissolved matter were uniformly mixed together, 0.1 parts by mass of a fragrance was then added thereto, the components were uniformly mixed and then crushed, and a foundation material was produced through a sieve. This foundation material was compression-molded on a metal plate to produce a powder foundation.

The powder foundation (topical preparation) containing the poly(3-hydroxybutyrate-co-3-hydroxyvalerate) resin particles of Example 2 was confirmed to be odorless, generate no squeaks, have excellent texture upon application (texture when the foundation was applied to skin), and be excellent in terms of adhesion to skin and easy spread on skin.

### [Preparation of Coating Material]

Two parts by mass of the poly(3-hydroxybutyrate-co-3-hydroxyvalerate) resin particles of Example 1 and 20 parts by mass of a commercially available acrylic aqueous glossy paint (manufactured by Kanpe Hapio Co., Ltd., trade name: SUPER HIT) were mixed together for three minutes and defoamed for one minute using a stirring and defoaming device, thereby obtaining a paint as a coating material. The obtained paint was applied onto an ABS resin (acrylonitrile butadiene styrene resin) plate using a coating device in which a blade having a clearance of 50 µm was set and then dried, thereby obtaining a coated film. The obtained coated film was measured using a gloss meter (manufactured by Horiba, Ltd., "GLOSS CHECKER IG-330"). The gloss (60°) of the coated film was one.

The present invention can be implemented in various other forms without departing from the spirit or main features thereof. Thus, the Examples described above are merely illustrative in all respects and must not be interpreted restrictively. The scope of the present invention is indicated by the claims and is not bound by the text of the description in any way. Furthermore, variations and modifications belonging to the equivalents of the claims are all within the scope of the present invention.

## Claims

1. A polyhydroxyalkanoate-based resin particle, wherein
a total remaining amount of an unsaturated fatty acid is 10 mass ppm or less.

2. A polyhydroxyalkanoate-based resin particle, wherein
a total remaining amount of crotonic acid is 10 mass ppm or less.

3. A polyhydroxyalkanoate-based resin particle, wherein
a total remaining amount of a pentenoic acid is 60 mass ppm or less.

4. The polyhydroxyalkanoate-based resin particle according to any one of claims 1 to 3,
wherein a polyhydroxyalkanoate-based resin contains a poly(3-hydroxybutyrate-co-3-hydroxyvalerate) resin.

5. The polyhydroxyalkanoate-based resin particle according to any one of claims 1 to 3, wherein
a volume average particle diameter is 1 µm or more and 40 µm or less.

6. The polyhydroxyalkanoate-based resin particle according to any one of claims 1 to 3, wherein
a moisture content is 0.2 mass% or more and 0.8 mass% or less.

7. A topical preparation comprising:
the resin particle according to any one of claims 1 to 3.

8. A coating material comprising:
the polyhydroxyalkanoate-based resin particle according to any one of claims 1 to 3.

9. A resin composition comprising:
the polyhydroxyalkanoate-based resin particle according to any one of claims 1 to 3.

10. An anti-blocking agent comprising:
the polyhydroxyalkanoate-based resin particle according to any one of claims 1 to 3.
